(19) Europäisches Patentamt
European Patent Office
Office européen des brevets

(11) Veröffentlichungsnummer: **0 408 759 A1**

(12) # EUROPÄISCHE PATENTANMELDUNG veröffentlicht nach Art. 158 Abs. 3 EPÜ

(21) Anmeldenummer: **90901086.0**

(22) Anmeldetag: **22.12.89**

(86) Internationale Anmeldenummer:
**PCT/SU89/00333**

(87) Internationale Veröffentlichungsnummer:
**WO 90/07479 (12.07.90 90/16)**

(51) Int. Cl.[5]: **C07B 39/00**, C07C 17/22

(30) Priorität: **30.12.88 SU 4622748**

(43) Veröffentlichungstag der Anmeldung:
**23.01.91 Patentblatt 91/04**

(84) Benannte Vertragsstaaten:
**CH DE GB IT LI**

(71) Anmelder: **ANDRIEVSKY, Alexandr Mikhailovich**
**ul. Sivashskaya, 4-3-100**
**Moscow, 113149(SU)**

Anmelder: **GORELIK, Mikhail Viktorovich**
**ul. Pervomaiskaya 46-60 Moskovskaya obl.**
**Dolgoprudny, 141700(SU)**

Anmelder: **AVIDON, Sergei Viktorovich**
**ul. Godovikova, 1-2-72**
**Moscow, 129085(SU)**

Anmelder: **NIKINOV, Valery Valentinovich**
**Starovatutinsky proezd, 1-86**
**Moscow, 129281(SU)**

Anmelder: **VOROZHTSOV, Georgy Nikolaevich**
**ul. Sadovaya-Spasskaya, 21-208**
**Moscow, 107078(SU)**

Anmelder: **LINKO, Roman Vladislavovich**
**Bulvar Filevsky, 12-169**
**Moscow, 121601(SU)**

Anmelder: **CHELYSHEVA, Olga Vyacheslavovna**
**ul. Nelidovskaya, 13-2-20**
**Moscow, 123363(SU)**

Anmelder: **POPLAVSKY, Alexandr Nikolaevich**
**ul. 3 Frunzenskaya, 6-22**
**Moscow, 119270(SU)**

Anmelder: **DJUMAEV, Kirill Mikhailovich**
**ul. B. Filevskaya, 55-2-27**
**Moscow, 121433(SU)**

(72) Erfinder: **ANDRIEVSKY, Alexandr Mikhailovich**
**ul. Sivashskaya, 4-3-100**
**Moscow, 113149(SU)**
Erfinder: **GORELIK, Mikhail Viktorovich**
**ul. Pervomaiskaya 46-60 Moskovskaya obl.**
**Dolgoprudny, 141700(SU)**
Erfinder: **AVIDON, Sergei Viktorovich**
**ul. Godovikova, 1-2-72**
**Moscow, 129085(SU)**
Erfinder: **NIKINOV, Valery Valentinovich**
**Starovatutinsky proezd, 1-86**
**Moscow, 129281(SU)**
Erfinder: **VOROZHTSOV, Georgy Nikolaevich**
**ul. Sadovaya-Spasskaya, 21-208**
**Moscow, 107078(SU)**
Erfinder: **LINKO, Roman Vladislavovich**
**Bulvar Filevsky, 12-169**
**Moscow, 121601(SU)**
Erfinder: **CHELYSHEVA, Olga Vyacheslavovna**
**ul. Nelidovskaya, 13-2-20**
**Moscow, 123363(SU)**
Erfinder: **POPLAVSKY, Alexandr Nikolaevich**
**ul. 3 Frunzenskaya, 6-22**
**Moscow, 119270(SU)**
Erfinder: **DJUMAEV, Kirill Mikhailovich**
**ul. B. Filevskaya, 55-2-27**
**Moscow, 121433(SU)**



Xerox Copy Centre

(74) Vertreter: **von Füner, Alexander, Dr. et al**
**Patentanwälte v. Füner, Ebbinghaus, Finck**
**Mariahilfplatz 2 & 3; 3**
**D-8000 München 90(DE)**

## (54) VERFAHREN ZUR HERSTELLUNG VON BROMIERTEN AROMATISCHEN UND HETEROZYKISCHEN DIE AKZEPTORGRUPPEN ENTHALTENDEN VERBINDUNGEN ZUSAMMENFASSUNG

Verfahren zur Herstellung von bromierten afomatischen und heterozyklischen die Akzeptor-Gruppen aufweisenden Verbindungen besteht darin, dass man die Bromierung aromatischer und heterozyklischer Verbindungen, die Akzeptor-Gruppen aufweisen, mit Brom oder mit Salzen der Bromwasserstoffsäure vornimmt, wobei man den Prozess in Gegenwart der Schwefelsäure oder des Oleums in Kombination mit Salpetersäure oder ihren Salzen bei einer Temperatur von 20 bis 120° C bei folgenden Molverhältnissen der Reagenzien je l Mol einer Ausgangsverbindung durchführt: Brom von 0,5 bis 3,2 oder Salz der Bromwasserstoffsäure von l,0 bis 7,0, Salpetersäure von 0,5 bis 3,0 oder ein Salz der Salpetersäure von l,0 bis 4,0. Schwefelsäure oder Oleum von 6,0 bis 70,0.

Die gewonnenen Verbindungen finden als Antipyrene, als Zwischenprodukte bei der Synthese von Farbstoffen, Pigmenten, Herbiziden und in anderen Bereichen Anwendung.

EPAA-39719.3

# VERFAHREN ZUR HERSTELLUNG VON BROMIERTEN AROMATISCHEN UND HETEROZYKLISCHEN AKZEPTORGRUPPEN ENTHALTENDEN VERBINDUNGEN

## Gebiet der Technik

Die vorliegende Erfindung bezieht sich auf das Gebiet der organischen Chemie, und insbesondere bezieht sich auf ein Verfahren zur Herstellung von bromierten aromatischen und heterozyklischen Akzeptor-Gruppen enthaltenden Verbindungen.

## Zugrundeliegender Stand der Technik

Bekannt sind verschiedene Verfahren zur Herstellung von bromierten aromatischen und heterozyklischen Verbindungen, die auf Bromierung aromatischer und heterozyklischer Verbindungen mit Molekularbrom in Gegenwart verschiedener Katalysatoren (Lewis-Säure, beispielsweise, komplexer stickstoffhaltiger Verbindungen und anderer) beruhen, beispielsweise, ein Verfahren, das die Umsetzung einer entsprechenden aromatischen Verbindung mit Molekularbrom in einem Lösungsmittel und in Gegenwart der Katalysatoren der Bromierung, Metallchloride, vorsieht. Als Lösungsmittel verwendet man Tetrachloräthan und Xylol (DE, C, 2835655).

Das genannte Verfahren zeichnet sich durch die Verwendung von Halogenmetallen als Katalysatoren aus, die bei ihren grosstechnischen Verwendung schwerverwertbare Abfälle bilden. Es ist ausserdem der Überschuss am Molekularbrom erforderlich, weil eine Hälfte von ihm in Form der Bromwasserstoffsäure verlorengeht. Unter Bedingungen des genannten Verfahrens ist es nicht möglich, die Bromierung aromatischer Verbindungen vorzunehmen, die Akzeptor-Gruppen enthalten.

Bekannt ist ein Verfahren der Bromierung aromatischer Verbindungen mit Molekularbrom im Medium konzentrierter Schwefelsäure in Gegenwart folgender Verbindungen: $RSO_4OAg$, worin

R = 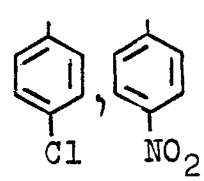 , $CF_3$

(Synthesis, 1978, 9,693-694).

Das genannte Verfahren setzt die Verwendung kostspieliger Verbindungen auf Ag-Grundlage (Silber) voraus. Ausserdem lassen sich die Verbindungen, die mehr als eine Akzeptor-Gruppe enthalten, mit Schwierigkeiten bromieren; dabei wird ein langwieriges Halten bei hohen Temperaturwerten angewendet. So wird, beispielsweise, das I,3-Dinitrobenzol innerhalb von I6 Stunden bei einer Temperatur von 90°C (Ausbeute beträgt 7I,0 Masse%) bromiert. Unter den beschriebenen Bedingungen entstehen keine Produkte mit einem höheren Bromierungsgrad.

Bekannt sind ebenfalls Verfahren zur Bromierung aromatischer und heterozyklischer Verbindungen mit Bromsalzen in Gegenwart von Oxydationsmitteln.

Beispielsweise, ein Verfahren zur Herstellung von chlorierten und bromierten oder jodierten aromatischer und heterozyklischer Verbindungen (Benzol, Anisol, Azetanilid, Salizylsäure, p-Methoxytoluol, Mesitylen, Durol, m-Xylol, Naphthalin verläuft durch Behandlung mit Chloriden, Bromiden oder Jodiden von Alkalisalzen, Erdalkali- oder Ammoniumsalzen in Gegenwart eines Oxydation-Reduktion-Paares, das aus der Gruppe gewählt ist: $Cu^{2+}/Cu^{+}$; $Fe^{3+}/Fe^{2+}$; $NO_3^-/NO_2^-$ (DE, C, 292908I).

Das genannte Verfahren wird durch die Prozessführung bei einem Druck von I bis 3 Bar und bei einer Temperatur von 30 bis 200°C charakterisiert. Das Verfahren lsst sich zum Bromieren von keine Akzeptor-Gruppen enthaltenden Verbindungen anwenden.

Darüber hinaus sind Verfahren zur Bromierung mit Molekularbrom in Gegenwart von nitrierenden Agenzien bekannt. Beispielsweise, ein Verfahren zur Halogenierung aromatischer Kohlenwasserstoffe in Gegenwart eines nitrierenden Gemisches folgender Zusammensetzung: $HNO_3$, $H_2SO_4$, $CH_3COOH$ (Nachrichten der Hochschulen der UdSSR, "Chemie und chemische Technologie", I969, Band 3, Ausgabe 5, S. 872-875). Das Vorhandensein in dem genannten Verfahren von Essigsäure in dem nitrierenden Gemisch unterdrückt die Möglichkeiten des Systems bei Bromierung aromatischer Verbindungen, die eine oder mehrere Akzeptor-Gruppen enthalten. So wurde das Nitrobrombenzol in einer niedrigen Ausbeute (33 Masse%) gewonnen, und das I,3-Dinitrobenzol lässt sich nicht sogar bei Erwärmung auf I20°C bromieren.

Bekannt ist ebenfalls ein Verfahren zur Bromierung im Medium konzentrierter Schwefelsäure, Oleums oder Fluorsulfonsäure mit Dibromisozyanursäure (Monatshefte für Chemie, I968, 99, 8I5; I969, I00, 42). Gemäss diesem Verfahren gelingt es, Bromierungsprodukte von Verbindungen zu gewinnen, die Akzeptor-Gruppen enthalten. So entsteht, beispielsweise, das I-Brom-3,5-dinitrobenzol mit einer Ausbeute von 86 Masse%.

Das genannte Verfahren zeichnet sich durch die Kompliziertheit der technologischen Prozessführung infolge der Verwendung der Dibromisozyanursäure als bromierendes Agens aus, die man aus Zyanursäure durch Bromieren derselben mit viermaligem Uberschuss an Brom in der Lösung des Lithiumhydroxid innerhalb von 24 Stunden gewinnt.

Offenbarung der Erfindung

Der Erfindung liegt die Aufgabe zugrunde, durch die Veränderung der Bedingungen der Prozessführung ein Verfahren zu entwickeln, das es ermöglicht, die Technologie zu vereinfachen, die Ausbeute an Endprodukt zu vergrössern und die Bromierung beliebiger aromatischer und heterozyklischer die Akzeptor-Gruppen enthaltenden Verbindungen vorzunehmen.

Die Aufgabe wird dadurch gelöst, dass man, erfindungsgemäss, im angemeldeten Verfahren zur Herstellung bromierter aromatischer und heterozyklischer die Akzeptor-Gruppen enthaltenden Verbindungen durch die Behandlung der aromatischen und heterozyklischen Verbindungen, die Akzeptor-Gruppen enthalten, mit einem bromierenden Agens in Gegenwart von Schwefelsäure oder Oleum unter Anfallen des Endproduktes, erfindungsgemäss, als bromierendens Agens Brom oder Salze der Bromwasserstoffsäure verwendet, wobei man den Prozess in Gegenwart der Schwefelsäure oder Oleum in Kombination mit Salpetersäure oder mit ihren Salzen bei einer Temperatur von 20 bis I20°C bei folgenden Molverhältnissen der Reagenzien je I Mol der Ausgangsverbindung: Brom 0,5-3,2 oder Salz der Bromwasserstoffsäure I,0-7,0, Salpetersäure 0,5-3,0 oder

Salz der Salpetersäure I,0-4,0, Schwefelsäure oder Oleum 6,0-70,0.

Das beanspruchte Verfahren ermöglicht es, die Technologie zu vereinfachen und die Ausbeute an Endprodukten (gegenüber dem bekannten Verfahren zur Bromierung unter Verwendung eines boomierenden Agens, Dibromisozyanursäure) zu vergrössern. Das beanspruchte Verfahren ermöglicht es, die Bromierung beliebiger aromatischer und heterozyklischer die Akzeptor-Gruppen enthaltenden Verbindungen vorzunehmen.

Beste Ausführungsform der Erfindung

Das erfindungsgemässe Verfahren wird wie folgt durchgeführt.

Aromatische oder heterozyklische Verbindungen, die Akzeptor-Gruppen enthalten, werden der Bromierung ausgesetzt. Als bromierendes Agens verwendet man Molekularbrom oder Salz der Bromwasserstoffsäure, dabei verläuft der Prozess in Gegenwart der Salpetersäure oder ihrer Salze und der Schwefelsäure oder des Oleums bei folgenden Molverhältnissen je I Mol der Ausgangsverbindung: Brom 0,5-3,2 oder Salz der Bromwasserstoffsäure I,0-7,0, Salpetersäure 0,5-3,0 oder Salz der Salpetersäure I,0-4,0, Schwefelsäure oder Oleum 6,0-70,0.

Die Prozessführung erfolgt bei einer Temperatur von 20 bis I20°C.

Die gewählten Bedingungen ermöglichen es, die Bromierung im Gemisch der Salpeter- und Schwefelsäure vorzunehmen. Bei einem Verhältnis des Broms oder des Salzes der Bromwasserstoffsäure unter den genannten Bereichen (0,5 und I,0) geht die Teaktion nicht zu Ende, und bei Verhältnissen oberhalb der angemeldeten Bereiche ist es nicht zweckmässig, den Prozess durchzuführen, kraft eines grossen Verbrauchs an Brom. Analog wurden die Verhältnisse der Salpetersäure und ihrer Salze gewählt. Die Menge der Schwefelsäure oder des Oleums wird durch die untere Grenze 6,0, die für Auflösung der Ausgangsverbindung erforderlich ist, und durch die obere Grenze 70,0, die für die vollständige Durchführung des Prozesses bei einer minimalen Temperatur von 20°C erforderlich ist, festgelegt. Zur Beschleunigung der Prozessführung wird die Erwärmung des Reaktionsgemisches (in Abhängigkeit von der Ausgangsverbindung) auf eine Temperatur nicht oberhalb I20°C vorgenommen.

Die Verwendung der Bromide anstelle von Brom bei Bromierung und anstelle der Salpetersäure ihrer Salze bei Bromierung beeinflusst wesentlich die Dauer des Reaktionsverlaufs und die Ausbeute an Endprodukt nicht.

Die Ausbeute an Endprodukt beträgt höchstens 99,5% Masse%, berechnet auf Ausgangsbrom.

Zur besseren Erläuterung der vorliegenden Erfindung werden folgende Beispiele für die Realisierung des angemeldeten Verfahrens angeführt.

Beispiel I

I,68 g (0,0I Mol) I,3-Dinitrobenzol löst man in I5 ml (0,26 Mol) Schwefelsäure (d = I,83) auf, man setzt 0,85 g (0,005 Mol) Brom, 0,5 ml (0,0I Mol) Salpetersäure (d = I,505) (Verhältnis $Br_2:HNO_3:H_2SO_4$ = 0,5:I,0:26,0 je I Mol der Ausgangsverbindung) zu und erwärmt man bei einer Temperatur von 65 bis 70°C innerhalb von 2 Stunden Die Reaktionslösung wird abgekühlt und auf Eis ausgegossen. Der Niederschlag wird abgefiltert, mit Wasser gewaschen und getrocknet. Man erhält 2,23 g (99,3 Masse%) 5-Brom-I,3-dinitrobenzol (farblose Kristalle aus 50%iger wässerige Essigsäure). Der Schmelzpunkt beträgt von 72 bis 73°C (Literaturangaben zufolge beträgt der Schmelzpunkt 73°C). $R_f$ = 0,45 (Benzol:Heptan = I:I).

Beispiel 2

3,0 g (0,0I8 Mol) I,3-Dinitrobenzol löst man in 50 ml Oleum (freies Schwefligsäurenhydrid 20 Masse%) auf, man setzt 4,32 g (0,027 Mol) Brom, I,0 ml (0,028 Mol) Salpetersäure (d = I,505) (Verhältnis $Br_2:HNO_3$:Oleum = I,5:I,3:56,0 je I Mol der Ausgangsverbindung) zu und erwärmt man bei einer Temperatur von 65°C innerhalb von 3 Stunden. Die Reaktionslösung wird abgekühlt und auf Eis ausgegossen. Der Niederschlag wird abgefiltert, mit Wasser gewaschen und getrocknet. Man erhält 8,05 g Gemisch der Bromierungsprodukte.

I,0 g Gemisch löst man in 50 ml einem Gemisch der Lösungsmittel Benzol:Heptan=I:I auf, trägt man auf eine

Säule mit Silikagel L 40/IOO auf und eluiert man mit dem gewonnenen Gemisch, indem die Fraktion mit $R_f$ = 0,5I gesammelt wird. Das Lösungsmittel wird eingedampft und man erhält 0,63 g (63 Masse%) 2,3,4-Tribrom-I,5-dinitrobenzol (farblose Kristalle aus 50%iger wässeriger Essigsäure), der Schmelzpunkt beträgt von I52 bis I53°C (Literaturangaben zufolge beträgt der Schmelzpunkt I53°C) 405 $[M]^+$.

Beispiel 3

3,0 g (0,0I8 Mol) I,3-Dinitrobenzol löst man in 50 ml Oleum (freies Schwefeligsäureanhydrid 25 Masse%) auf, man setzt 9,3I g (0,058 Mol) Brom und tropfenweise I,5 ml (0,034 Mol) Salpetersäure (d = I,505) (Verhältnis $Br_2$:$HNO_3$:Oleum = 3,2:I,9:57,0 je I Mol der Ausgangsverbindung) zu. Man erwärmt bei einer Temperatur von 70 bis 75°C innerhalb von 5 Stunden. Dann wird die Reaktionslösung abgekühlt und auf Eis ausgegossen. Der Niederschlag wird abgefiltert und getrocknet. Man erhält 8,9 g Produkte der Bromierung, die man aus Tetrachlorkohlenstoff umkristallisiert, man erhält 6,69 g (67,3 Masse%) Hexabrombenzol (farblose Kristalle aus Xylol), der Schmelzpunkt beträgt von 305 bis 308°C (Literaturangaben zufolge beträgt der Schmelzpunkt von 324 bis 326°C).

Beispiel 4

IO,0 g (0,06 Mol) 3-Nitrobenzoesäure löst man in 40 ml (0,7 Mol) Schwefelsäure (d = I,83) auf, man setzt I,54 ml (0,03 Mol) Brom zu, man lässt die Temperatur bis 70°C steigen und man setzt langsam tropfenweise unter Vermischen I,26 ml (0,03 Mol) Salpetersäure (d = I,505) (Verhältnis $Br_2$:$HNO_3$:$H_2SO_4$ = 0,5:0,5:I2,0 je I Mol der Ausgangsverbindung) zu, und man lässt bei dieser Temperatur innerhalb von 4 Stunden stehen. Die Reaktionslösung wird abgekühlt, auf Eis ausgegossen, der Niederschlag wird abgefiltert. Man erhält I4,2 g (96,2 Masse%) 3-Nitro-5-brombenzoesäure (farblose Kristalle aus 50%iger Essigsäure), der Schmelzpunkt beträgt von I57 bis I59°C) (Literaturangaben zufolge beträgt der Schmelzpunkt von I58 bis I6I°C).

Beispiel 5

I0,0 g (0,034 Mol) 2,7-Dinitrophenanthrenchinon löst man in I20 ml (2,26 Mol) Schwefelsäure (d = I,83) auf, man setzt I2,4 g (0,08 Mol) Brom, 4,0 ml (0,09 Mol) Salpetersäure (d = I,505) (Verhältnis $Br_2:HNO_3:H_2SO_4$ = 2,4:2,6:66,0 je I Mol der Ausgangsverbindung) zu, erwärmt man auf eine Temperatur von 50 bis 55°C und lässt man innerhalb von 2 Stunden stehen. Die Reaktionslösung wird abgekühlt, auf Eis ausgegossen, der Niederschlag wird abgefiltert. Man erhält 9,6 g Reaktionsprodukt, das man aus Essigsäure umkristallisiert. Man erhält 8,0 g (62,5 Masse%) 4-Brom-2,7-dinitrophenanthrenchinon (gelbe Kristalle aus Essigsäure). Der Schmelzpunkt beträgt von 220,5 bis 22I,5°C, $R_f$ = 0,45 (Benzol:Azeton = 7:I).

Gefunden, %: C 44,27, 44,49: H I,90, I,58; N 7,70, 7,62: Br 2I,32, 22,II. $C_{14}H_5N_2O_6Br$.

Berechnet, %: C 44,38; H I,33; N 7,43; Br 2I,22.

IR-Spektrum, $cm^{-1}$/KCl:I700/C=O), I340 und I5I5 ($NO_2$).

Beispiel 6

8,0 g (0,027 Mol) 2-5-Dinitrophenanthrenchinon löst man in I00 ml (I,74 Mol) Schwefelsäure auf, man setzt I2,4 g (0,08 Mol) Brom zu und erwärmt man auf eine Temperatur von 40 bis 45°C. Dann setzt man tropfenweise 2,0 ml (0,045 Mol) Salpetersäure (d = I,505) zu, (Verhältnis $Br_2:HNO_3:H_2SO_4$) 2,9:I,I:58,I je I Mol der Ausgangslösung). Man lässt bei dieser Temperatur innerhalb von 2 Stunden und dann weitere 0,5 Stunden bei einer Temperatur von 55°C stehen. Die Reaktionslösung wird abgekühlt, auf Eis ausgegossen, der Niederschlag wird abgefiltert. Nach der Umkristallisation aus Essigsäure erhält man 5,4 g (53,I Masse%) 2-Brom-4,7-dinitrophenanthrenchinon (gelbe Kristalle aus Essigsäure). Der Schmelzpunkt beträgt von 253,4 bis 254,5°C; $R_f$ = 0,60 (Benzol:Azeton = 7:I).

Gefunden, %: 44,20, 44,30; H I,9I, I,90; N 7,5I; 7,40; Br 2I,I9, 20,92. $C_{14}H_5N_2O_6Br$.

Berechnet, %: C 44,56; H I,33; N 7,43; Br 2I,22.

IR-Spektrum, $cm^{-1}$ /KCl:I685 (C=O), I350 und I525 ($NO_2$).

Beispiel 7

I0,0 g (0,03 Mol) 2,4,8-Trinitro-6(5H)-phenantridinon

löst man in I00 ml (I,74 Mol) Schwefelsäure (d = I,83) auf, man setzt 2,58 g (0,0I6 Mol) Brom zu und erwärmt man auf eine Temperatur von 75°C. Dann setzt man tropfenweise I,4 ml (0,032 Mol) Salpetersäure (d = I,55) (Verhältnis $Br_2:HNO_3:H_2SO_4$ = 3,0:I,7:64,0 je I Mol der Ausgangsverbindung) zu. Die Reaktionslösung wird bei einer Temperatur von 85 bis 90°C innerhalb von 4 Stunden vermischt. Nach der Abkühlung wird sie auf Eis ausgegossen, der Niederschlag wird abgefiltert, mit Wasser gewaschen und getrocknet. Man erhält I0,06 g (82,0 Masse%) I0-Brom-2,4,8-trinitro-6(5H)-phenantridinon. Der Schemlzpunkt beträgt von 226 bis 228°C, $R_f$ = 0,56 (Azeton:Benzol = I:7).

Gefunden, %: C 38,8, H I,2; N I3,5; Br I9,4.

$C_{I3}H_5N_4O_7Br$.

Berechnet, %: C 38,I; H I,2; N I3,7; Br I9,6.

IR-Spektrum, $cm^{-I}$ /KCl:I700 (C=O), I350 und I540 ($NO_2$), 3I00 und 3200 (NH).

Beispiel 8

2,0 g (0,0I Mol) 2,4-Dinitrochlorbenzol löst man in 40 ml (0,7 Mol) Schwefelsäure (d = I,83) auf, man setzt 2,4 g (0,0I5 Mol) Brom, 0,65 ml (0,0I Mol) Salpetersäure (d = I,505) (Verhältnis $Br_2:HNO_3:H_2SO_4$ = I,5:I,5:70,0 je I Mol der Ausgangsverbindung) zu und vermischt man bei Raumtemperatur innerhalb von 63 Stunden. Die Reaktionsmasse wird auf I00 g Eis ausgegossen, der Niederschlag abgefiltert, mit Wasser gewaschen und getrocknet. Man erhält 2,38 g (84,8 Masse%) 2-Brom-4,6-dinitrochlorbenzol (hellgelbe Kristalle aus 70%iger wässeriger Essigsäure). Der Schmelzpunkt beträgt von 6I bis 62°C (Literaturangaben zufolge beträgt der Schmelzpunkt 62°C), $R_f$ = 0,43 (Benzol:Hexan = I:I).

Beispiel 9

I,0 g (0,005 Mol) 2-4-Dintrochlorbenzol löst man in 30 ml (0,35 Mol) Schwefelsäure (d = I,62) auf, man setzt 0,8 g (0,005 Mol) Brom, 0,43 ml (0,0I Mol) Salpetersäure (d = I,505) (Verhältnis $Br_2:HNO_3:H_2SO_4$ = I,0:2,0:70,0 je I Mol der Ausgangsverbindung) zu und erwärmt man bei einer Temperatur von I20°C. Die Reaktionslösung wird bei dieser Temperatur innerhalb von 55 Stunden vermischt, dann

abgekühlt und auf I50 g Eis ausgegossen. Der Niederschlag wird abgefildert, mit Wasser gewaschen und getrocknet. Man erhält 2I,28 g (90,9 Masse%) 2-Brom-2,6-dinitrochlorbenzol.

Beispiel IO

60,75 g (0,3 Mol) 2,4-Dinitrochlorbenzol löst man in I00 ml (I,8 Mol) Schwefelsäure (d = I,83) auf, man setzt 26,4 g (0,I65 Mol) Brom und tropfenweise I3,0 ml (0,3 Mol) Salpetersäure (d = I,505) (Verhältnis $Br_2:HNO_3:H_2SO_4$ = 0,5:I,0:6,0 je I Mol der Ausgangsverbindung) zu, indem die Temperatur in einem Bereich von 65 bis 70°C unterhalten wird. Dann vermischt man innerhalb von 25 Stunden bei einer Temperatur von 65°C, kühlt man die Reaktionslösung ab und giesst man auf 250 g Eis aus, der Niederschlag wird abgefiltert, mit Wasser gewaschen und getrocknet. Man erhält 83,03 (99,7 Masse%) 2-Brom-4,6-dinitrochlorbenzol.

Beispiel II

In 42 ml (0,75 Mol) Schwefelsäure (d = I,83) löst man 2,63 g (0,0I3 Mol) 2-4-Dinitrochlorbenzol auf, man setzt I,I2 g (0,007 Mol) Brom und tropfenweise I,70 ml (0,039 Mol) Salpetersäure (d = I,505) (Verhältnis $Br_2:HNO_3:H_2SO_4$ = 0,5:3,0:60,0 je I Mol der Ausgangsverbindung) zu, indem die Temperatur in einem Bereich von 45 bis 50°C unterhalten wird. Dann wird das Vermischen innerhalb von 6 Stunden bei einer Temperatur von 65 bis 70°C vorgenommen. Nach der Ausscheidung des Produktes erhält man 3,64 g (99,5 Masse%) 2-Brom-4,6-dinitrochlorbenzol.

Beispiel I2

0,5 g (0,0023 Mol) 2,4-Dichlor-5-nitrobenzonitril löst man in 9 ml (0,I56 Mol) Schwefelsäure (d = I,83) auf, man setzt 0,I ml (0,0023 Mol) Salpetersäure (d = I,5I3) und 0,062 ml (0,00I2 Mol) Brom zu. (Verhältnis $Br_2:HNO_3:H_2SO_4$ = 0,5:I,0:68,0 je I Mol der Ausgangsverbindung). Man lässt die Temperatur auf 60°C steigen und vermischt man innerhalb von 6 Stunden. Dann wird die Reaktionsmasse abgekühlt und auf Eis ausgegossen. Der ausgefallene Niederschlag wird abgefiltert, mit Wasser gewaschen und getrocknet. Man erhält 0,6I g (84,7 Masse%)

3-Brom-2,4-dichlor-5-nitrobenzamid, der Schmelzpunkt beträgt von I73 bis I75°C, 3I4 $[M]^+$. $R_f$ = 0,4 (Benzol: Azeton = 3:I).

Beispiel I3

Einer Lösung aus I,68 g (0,0I Mol) I,3-Dinitrobenzol in I5 ml Schwefelsäure (d = I,83) setzt man 0,85 g (0,005 Mol) Brom und I,5I g (0,0I5 Mol) Kaliumnitrat (Verhältnis $Br_2:KNO_3:H_2SO_4$ = 0,5:I,5:26,0 je I Mol der Ausgangsverbindung) zu. Die Reaktionsmasse wird auf eine Temperatur von 65°C erwärmt und innerhalb von 2,5 Stunden stehengelassen, dann abgekühlt und auf Eis ausgegossen. Der Niederschlag wird abgefiltert, mit Wasser, mit einer 5%iger Natriumkarbonatlösung und mit warmem Wasser gewaschen und getrocknet. Man erhält 2,I6 g (87,5 Masse%) 5-Brom-I,3-dinitrobenzol.

Beispiel I4

Einer Lösung aus 3,0 g (0,0I8 Mol) I,3-Dinitrobenzol in 50 ml Oleum (freies Schwefligsäureanhydrid 25 Masse%) setzt man 9,3 g (0,057 Mol) Brom und 5,I8 g (0,054 Mol) Kaliumnitrat (Verhältnis $Br_2:KNO_3$:Oleum = 3,2:3,0:56,0 je I Mol der Ausgangsverbindung) zu. Man erwärmt die Reaktionsmasse auf eine Temperatur von 80°C und lässt man innerhalb von 7,5 Stunden stehen, wäscht man mit Wasser, mit einer 5%igen Natriumkarbonatlösung und mit warmem Wasser und trocknet man, dann kristallisiert man aus Tetrachlorkohlenstoff aus und man erhält 5,2 g (52,3 Masse%) Hexabrombenzol.

Beispiel I5

Einer Lösung aus I0,0 g (0,06 Mol) 3-Nitrobenzoesäure in 40 ml Schwefelsäure (d = I,83) setzt man 4,78 g (0,03 Mol) Brom und 6,06 g (0,06 Mol) Kaliumnitrat 9(Verhältnis $Br_2:KNO_3:H_2SO_4$ = 0,5:I,0:I2,0 je I Mol der Ausgangsverbindung) zu. Man erwärmt die Reaktionsmasse auf eine Temperatur von 75°C und lässt innerhalb von 5 Stunden stehen. Das Produkt wird analog Beispiel I3 ausgeschieden, man erhält I3,35 g (90,5 Masse%) 5-Brom-3-nitrobenzoesäure.

Beispiel I6

Einer Lösung aus 2,0 g (0,0I Mol) 2,4-Dinitrochlor-

benzol in 40 ml Schwefelsäure (d = I,83) setzt man 32,4 g (0,0I5 Mol) Brom und 2,28 g (0,023 Mol) Kaliumnitrat (Verhältnis $Br_2$:$KNO_2$:$H_2SO_4$ = I,5:2,25:70,0 je I Mol der Ausgangsverbindung) zu. Die Reaktionsmasse wird bei einer Temperatur von 20°C innerhalb von 75 Stunden stehengelassen. Das Produkt wird analog Beispiel I3 ausgeschieden, man erhält 2,I2 g (75,I Masse%) 2-Brom-4,6-dinitrochlorbenzol.

Beispiel I7

Einer Lösung aus I,0 g (0,005 Mol) 2,4-Dinitrochlorbenzol in 30 ml (0,35 Mol) Schwefelsäure (d = I,62) setzt man 0,8 g (0,005 Mol) Brom und I,5I g (0,0I5 Mol) Kaliumnitrat (Verhältnis $Br_2$:$KNO_3$:$H_2SO_4$ = I,0:3,0:70,0 je I Mol der Ausgangsverbindung) zu. Die Reaktionsmasse wird auf eine Temperatur von I20°C erwärmt und innerhalb von 60 Stunden stehengelassen. Das Produkt wird analog Beispiel I3 ausgeschieden, man erhält I,I5 g (82,0 Masse%) 2-Brom-4,6-dinitrochlorbenzol.

Beispiel I8

Einer Lösung aus 60,75 g (0,3 Mol) 2,4-Dinitrochlorbenzol in I00 ml Schwefelsäure (d = I,83) setzt man 26,4 g (0,05 Mol) Brom und 38,2 g (0,45 Mol) Natriumnitrat (Verhältnis $Br_2$:$NaNO_3$:$H_2SO_4$ = 0,5:I,5:6,0 je I Mol der Ausgangsverbindung) zu. Man erwärmt die Reaktionsmasse auf eine Temperatur von 70°C und lässt innerhalb von 35 Stunden stehen. Das Produkt wird analog Beispiel I3 ausgeschieden, man erhält 80,73 g (95,6 Masse%) 2-Brom-4,6-dinitrochlorbenzol.

Beispiel I9

Einer Lösung aus 2,63 g (0,0I3 Mol) 2,4-Dinitrochlorbenzol in 40 ml Schwefelsäure (d = I,83) setzt man I,I2 g (0,007 Mol) Brom und 5,3 g (0,052 Mol) Kaliumnitrat (Verhältnis $Br_2$:$KNO_3$:$H_2SO_4$ = 0,5:4,0:60,0 je I Mol der Ausgangsverbindung) zu. Man erwärmt die Reaktionsmasse auf eine Temperatur von 70°C und lässt innerhalb von 6 Stunden stehen. Das Produkt wird analog Beispiel I3 ausgeschieden. Man erhält 3,62 g (98,9 Masse%) 2-Brom-4,6-dinitrochlorbenzol.

Beispiel 20

Einer Lösung aus I,68 g (0,0I Mol) I,3-Dinitrobenzol

in I5 ml Schwefelsäure (d = I,83) setzt man I,2 g (0,0I Mol) Kaliumbromid und 0,5 ml (0,0I Mol) Salpetersäure (d = I,505) (Verhältnis $KBr_2:HNO_3:H_2SO_4$ = I,0:I,0:26,0 je I Mol der Ausgangsverbindung) zu. Man erwärmt die Reaktionsmasse auf eine Temperatur von 65°C und lässt innerhalb von 2 Stunden stehen, dann kühlt man ab und giesst man auf Eis aus. Der Niederschlag wird abgefiltert, mit Wasser, mit einer 5%iger Natriumkarbonatlösung und mit warmem Wasser gewaschen und getrocknet. Man erhält 2,20 g (89,I Masse%) 5-Brom-I,3-dinitrobenzol.

Beispiel 2I

Einer Lösung aus 3,0 g (0,0I8 Mol) I,3-Dinitrobenzol in 50 ml Oleum (freies Schwefligsäureanhydrid 25 Masse%) setzt man I4,87 g (0,I26 Mol) Kaliumbromid und I,5 ml (0,036 Mol) Salpetersäure (d = I,505) (Verhältnis $KBr_2:HNO_3$:Oleum = 7,0:2,0:57,0 je I Mol der Ausgangsverbindung) zu. Man erwärmt die Reaktionsmasse auf eine Temperatur von 75°C und lässt innerhalb von 6 Stunden stehen, dann kühlt man ab und giesst man auf Eis aus. Der Niederschlag wird abgefiltert, mit Wasser, mit einer 5%iger Natriumkarbonatlösung und mit warmem Wasser gewaschen und getrocknet, dann aus Tetrachlorkohlenstoff umkristallisiert, man erhält 7,05 g (70,9 Masse%) Hexabrombenzol.

Beispiel 22

Einer Lösung aus I0,0 g (0,06 Mol) 3-Nitrobenzoesäure in 40 ml Schwefelsäure (d = I,83) setzt man 7,I4 g (0,06 Mol) Kaliumbromid und I,26 ml (0,03 Mol) Salpetersäure (d = I,505) (Verhältnis $KBr_2:HNO_3:H_2SO_4$ = I,0:0,5:I2,0 je I Mol der Ausgangsverbindung) zu. Man erwärmt die Reaktionsmasse auf eine Temperatur von 70°C und lässt innerhalb von 4 Stunden stehen. Das Produkt wird analog Beispiel 20 ausgeschieden, man erhält I4, 5 g (98,I Masse%) 5-Brom-3-nitrobenzoesäure.

Beispiel 23

Einer Lösung aus 2,0 g (0,0I Mol) 2,4-Dinitrochlorbenzol in 40 ml Schwefelsäure (d = I,83) setzt man 3,58 g (0,03 Mol) Kaliumbromid und 0,65 ml (0,0I5 Mol) Salpetersäure (d = I,505) (Verhältnis $KBr:HNO_3:H_2SO_4$ =

3,0:I,5:70,0 je I Mol der Ausgangsverbindung) zu. Die Reaktionsmasse lässt man innerhalb von 70 Stunden bei einer Temperatur von 20°C stehen. Das Produkt wird analog Beispiel 20 ausgeschieden, man erhält 2I4 g (74,0 Masse%) 2-Brom-4,6-dinitrochlorbenzol.

Beispiel 24

Einer Lösung aus I,0 g (0,005 Mol) 2,4-Dinitrochlorbenzol in 30 ml (0,35 Mol) Schwefelsäure (d = I,62) setzt man I,2 g (0,0I Mol) Kaliumbromid und 0,43 ml (0,0I Mol) Salpetersäure (d = I,505) (Verhältnis $KBr:HNO_3:H_2SO_4$ = 2,0:2,0:70,0 je I Mol der Ausgangsverbindung) zu. Man erwärmt die Reaktionsmasse auf eine Temperatur von I20°C und lässt innerhalb 320 ausgeschieden, man erhält I,30 g (92,4 Masse%) 2-Brom-4,6- dinitrochlorbenzol.

Beispiel 25

Einer Lösung aus 60,75 g (0,3 Mol) 2,4-Dinitrochlorbenzol in I00 ml Schwefelsäure (d = I,83) setzt man 34,0 g (0,3 Mol) Natriumbromid und I3,0 ml (0,3 Mol) Salpetersäure (d = I,505) (Verhältnis $NaBr:HNO_3:H_2SO_4$ = I,0:I,0:6,0 je I Mol der Ausgangsverbindung) zu. Man erwärmt die Reaktionsmasse auf eine Temperatur von 70°C und lässt innerhalb von 25 Stunden stehen. Das Produkt wird analog Beispiel 20 ausgeschieden, man erhält 83,43 g (98,8 Masse%) 2-Brom-4,6-dinitrochlorbenzol.

Beispiel 26

Einer Lösung aus 2,63 g (0,0I3 Mol) 2,4-Dinitrochlorbenzol in 40 ml Schwefelsäure (d = I,83) setzt man I,55 g (0,0I3 Mol) Kaliumbromid und I,7 ml (0,039 Mol) Salpetersäure (d = I,505) (Verhältnis $KBr:HNO_3:H_2SO_4$ = I,0:3,0:60,0 je I Mol der Ausgangsverbindung) zu. Die Reaktionsmasse wird auf eine Temperatur von 70°C erwärmt und innerhalb von 6 Stunden stehengelassen. Das Produkt wird analog Beispiel 20 ausgeschieden. Man erhält 3,64 g (99,5 Masse%) 2-Brom-4,6-dinitrochlorbenzol.

Beispiel 27

Einer Lösung aus I,68 g (0,0I Mol) I,3-Dinitrobenzol in I5 ml Schwefelsäure (d = I,83) setzt man I,2 g (0,0I Mol) Kaliumbromid und I,5I g (0,0I5 Mol) Kaliumnitrat (Verhältnis $KBr:KNO_3:H_2SO_4$ = I,0:I,5:26,0 je I Mol

der Ausgangsverbindung) zu. Man erwärmt die Reaktionsmasse auf eine Temperatur von 65°C und lässt innerhalb von 2,5 Stunden stehen, dann kühlt man ab und giesst man auf Eis aus. Der Niederschlag wird abgefildert, mit Wasser, mit einer 5%iger Natriumkarbonatlösung und mit warmem Wasser gewaschen und getrocknet. Man erhält 2,I4 g (86,7 Masse%) 5-Brom-I,3-dinitrobenzol.

Beispiel 28

Einer Lösung aus 3,0 g (0,0I8 Mol) I,3-Dinitrobenzol in 50 ml Oleum (freies Schwefligsäureanhydrid 25 Masse%) setzt man I4,87 g (0,I26 Mol) Kaliumbromid und 5,I8 g (0,054 Mol) Kaliumnitrat (Verhältnis $KBr:KNO_3$::Oleum = 7,0:3,0:57,0 je I Mol der Ausgangsverbindung) zu. Man erwärmt die Reaktionsmasse auf eine Temperatur von 80°C und lässt innerhalb von 8 Stunden stehen, dann kühlt man ab und giesst man auf Eis aus. Der Niederschlag wird abgefiltert, mit Wasser, mit einer 5%iger Natriumkarbonatlösung und mit warmem Wasser gewaschen und getrocknet, dann aus Tetrachlorkohlenstoff umkristallisiert, man erhält 5,08 g (5I,5 Masse%) Hexabrombenzol.

Beispiel 29

Einer Lösung aus I0,0 g (0,06 Mol) 3-Nitrobenzoesäure in 40 ml Schwefelsäure (d = I,83) setzt man 7,I4 g (0,06 Mol) Kaliumbromid und 6,06 g (0,06 Mol) Kaliumnitrat (Verhältnis $KBr:KNO_3:H_2SO_4$ = I,0:I,0:I2,0 je I Mol der Ausgangsverbindung) zu. Man erwärmt die Reaktionsmasse auf eine Temperatur von 75°C und lässt innerhalb von 5 Stunden stehen. Das Produkt wird analog Beispiel 27 ausgeschieden, man erhält I3,2 g (90,2 Masse%) 5-Brom-3-nitrobenzoesäure.

Beispiel 30

Einer Lösung aus 2,0 g (0,0I Mol) 2,4-Dinitrochlorbenzol in 40 ml Schwefelsäure (d = I,83) setzt man 3,58 g (0,03 Mol) Kaliumbromid und 2,27 g (0,023 Mol) Kaliumnitrat (Verhältnis $KBr:KNO_3:H_2SO_4$ = 3,0:2,23:70,0 je I Mol der Ausgangsverbindung) zu.

Die Reaktionsmasse wird innerhalb von 75 Stunden bei einer Temperatur von 20°C stehengelassen. Das Produkt wird analog Beispiel 27 ausgeschieden, man erhält 2,03 g (73,0 Masse%) 2-Brom-3,6-dinitrochlorbenzol.

Beispiel 3I

Einer Lösung aus I,0 g (0,005 Mol) 2,4-Dinitrochlorbenzol in 30 ml (0,35 Mol) Schwefelsäure (d = I,62) setzt man I,2 g (0,0I Mol) Kaliumbromid und I,5I g (0,0I5 Mol) Kaliumnitrat (Verhältnis KBr:$KNO_3$:$H_2SO_4$ = 2,0:3,0:70,0 je I Mol der Ausgangsverbindung) zu. Man erwärmt die Reaktionsmasse auf eine Temperatur von I20°C und lässt innerhalb von 57 Stunden stehen. Das Produkt wird analog Beispiel 27 ausgeschieden, man erhält I,II g (79,9 Masse%) 2-Brom-4,6-dinitrochlorbenzol.

Beispiel 32

Einer Lösung aus 60,75 g (0,3 Mol) 2,4-Dinitrochlorbenzol in I00 ml Schwefelsäure (d = I,83) setzt man 34,0 g (0,3 Mol) Natriumbromid und 38,24 g (0,45 Mol) Natriumnitrat (Verhältnis NaBr:$NaNO_3$:$H_2SO_4$ = I,0:I,5:6,0 je I Mol der Ausgangsverbindung) zu. Man erwärmt die Reaktionsmasse auf eine Temperatur von 70°C und lässt innerhalb von 35 Stunden stehen. Das Produkt wird analog Beispiel 27 ausgeschieden, man erhält 79,05 g (93,6 Masse%) 2-Brom-4,6-dinitrochlorbenzol.

Beispiel 33

Einer Lösung aus 2,63 g (0,0I3 Mol) 2,4-Dinitrochlorbenzol in 40 ml Schwefelsäure (d = I,83) setzt man I,55 g (0,0I3 Mol) Kaliumbromid und 5,3 g (0,052 Mol) Kaliumnitrat (Verhältnis KBr:$KNO_3$:$H_2SO_4$ = I,0:4,0:60,0 je I Mol der Ausgangsverbindung) zu. Man erwärmt die Reaktionsmasse auf eine Temperatur von 70°C und lässt innerhalb von 7 Stunden stehen. Das Produkt wird analog Beispiel 27 ausgeschieden.

Man erhält 3,58 g (97,8 Masse%) 2-Brom-4,6-dinitrochlorbenzol.

Beispiel 34

In 40 ml (0,7 Mol) Schwefelsäure (d = I,83) löst man 3,0 g (0,0I2 Mol) 2,4-Dinitrobrombenzol auf, man setzt 0,98 g (0,006 Mol) Brom und tropfenweise 0,52 ml (0,0I2 Mol) Salpetersäure (d = I,505) (Verhältnis $Br_2$:$HNO_3$:$H_2SO_4$ = 2,0:I,0:58,3 je I Mol der Ausgangsverbindung) zu, indem die Temperatur in einem Bereich von 60 bis 65°C unterhalten wird. Dann wird das Vermischen innerhalb von 7 Stunden bei einer Temperatur von 75°C vorgenommen, die Reaktionslösung abgekühlt und auf Eis ausgegossen, der Niederschlag wird abgefiltert, mit Wasser gewaschen und getrocknet. Man erhält 3,85 (87,5 Masse%) I,2-Dibrom-3,5-dinitrobenzol. Der Schmelzpunkt beträgt 7I°C (aus 70%iger wässeriger Essigsäure) (Literaturangaben zufolge beträgt der Schmelzpunkt 7I°C), 326 $[M]^+$.

Beispiel 35

I3,0 g (0,07 Mol) 2,4-Dinitrofluorbenzol löst man in I90 ml (3,53 Mol) Schwefelsäure (d = I,84) auf, man setzt 5,6 g (0,035 Mol) Brom und tropfenweise 3,48 ml (0,08 Mol) Salpetersäure (d = I,505) (Verhältnis $Br_2:HNO_3:H_2SO_4$ = 2,0:I,I:50,4 je I Mol der Ausgangsverbindung) zu, indem man die Temperatur in einem Bereich von 65 bis 70°C unterhält. Dann wird das Vermischen innerhalb von IO Stunden bei einer Temperatur von 80°C vorgenommen, die Reaktionslösung abgekühlt und auf Eis ausgegossen; der Niederschlag wird abgefiltert, mit Wasser gewaschen und getrocknet. Man erhält I8,0 g (97,0 Masss%) 2-Brom-4,6-dinitrofluorbenzol. Der Schmelzpunkt beträgt 70°C (aus 80%iger wässeriger Essigsäure ) (Literaturangaben zufolge beträgt der Schmelzpunkt 69°C), 265 $[M]^+$.

Beispiel 36

In 40 ml (0,7 Mol) Schwefelsäure (d = I,83) löst man 3,0 g (0,0I4 Mol) I,3-Bis-(trifluormethyl)-benzol auf, man setzt I,I2 g (0,007 Mol) Brom und tropfenweise 0,6I ml (0,0I4 Mol) Salpetersäure (d = I,505) (Verhältnis $Br_2:HNO_3:H_2SO_4$ = 0,5:I,0:50,0 je I Mol der Ausgangsverbindung) zu, indem man die Temperatur von 55°C unterhält. Dann wird bei dieser Temperatur das Vermischen innerhalb von 3 Stunden vorgenommen, die Reaktionslösung abgekühlt und auf Eis ausgegossen. Die organische Schicht wird abgeschieden und die wässerige Lösung mit Chloroform extrahiert. Das Produkt und der Extrakt werden mit Wasser, mit einer I0%igen Natriumkarbonatlösung und wieder mit Wasser gewaschen. Der Extrakt wird über $Mg_2SO_4$ getrocknet, das Lösungsmittel wird abdestilliert und das Produkt bei einer Temperatur von I5I bis I52°C (750 Torr) abdestilliert. Man erhält 3,25 g (79,2 Masse%) I,3-Bis-(trifluormethyl)--5-brombenzol.

Beispiel 37

5,0 g (0,02I Mol) I,3-Benzoldisulfonsäure löst man in 20 ml Oleum (freies Schwefligsäurenahydrid IO Masse%) (0,39 Mol) auf, man setzt I,76 g (0,0II Mol) Brom und tropfenweise 0,92 ml (0,022 Mol) Salpetersäure (d = I,52) (Verhältnis $Br_2:HNO_3:H_2SO_4$ = 0,5:I,0:I8,0 je I Mol der Ausgangsverbindung) zu, indem man die Temperatur von 65°C unterhält. Bei dieser Temperatur wird das Vermischen innerhalb von 3 Stunden vorgenommen, die Reaktionslösung bis 5-IO°C gekühlt und der ausgefallene Niederschlag des Produktes wird abgefiltert. Das Produkt wird durch Erwärmung mit Uberschuss an Phosphorpentachlorid in Disulfochlorid umgewandelt. Die Ausbeute an 5-Brom-I,3-benzol-disulfochlorid beträgt 4,88 g (65,7 Masse%), der Schmelzpunkt beträgt von 98 bis 99°C (Literaturangaben zufolge beträgt der Schmelzpunkt 99°C). Gefunden, %: C 20,26; H 0,98; Br 22,5I; Cl 20,0I; O I7,95; S I8,27. $C_6H_3BrCl_2O_4S_2$.
Berechnet, %: C 20,34; H 0,85; Br 22,60; Cl 20,06; O I8,08, S I8,08.

Beispiel 38

Einer Lösung aus I4,3 g (0,05 Mol) 3,8-Dinitro-6H-dibenzo- [b,d] - pyran-6-on in 200 ml (3,47 Mol) Schwefelsäure (d = I,83) setzt man 6 ml (0,I2 Mol) Brom zu und vermischt man bei Raumtemperatur innerhalb I Stunde. Dann setzt man 6 ml (0,I4 Mol) Salpetersäure (d = I,52) (Verhältnis $Br_2:HNO_3:H_2SO_4$ = 2,3:2,8:70,0 je I Mol der Ausgangsverbindung) zu, man erwärmt auf eine Temperatur von 35 bis 40°C und vermischt bei dieser Temperatur innerhalb von 4 Stunden. Die Reaktionsmasse kühlt man ab und giesst auf Eis aus. Der ausgefallene Niederschlag wird abgefiltert, mit kaltem Wasser gewaschen und getrocknet. Man erhält nach der Umkristallisation aus der Essigsäure I6,5 g (90,4 Masse%) 2-Brom-3,8-dinitro-6H-dibenzo- [b,d]-pyran-6-on. Der Schmelzpunkt beträgt von 206 bis 207°C, $R_f$ = 0,7 (Azeton:Benzol = I:IO).

Berechnet, %: C 42,74; H I,37; N 7,67; Br 2I,92; $C_{I3}H_5N_2O_6Br$.

Gefunden, %: C 43,0I; 42,90; H I,29; I,38; N 7,77; 7,54; Br 22,II, 22,29; IR-Spektrum, (KBr) $cm^{-I}$: 3450, 3040, I770 (C=O), I630 und I540 ($NO_2$), I475, I365 ($NO_2$).

Industrielle Verwertbarkeit

Das beanspruchte Verfahren kann bei der Herstellung von bromhaltigen aromatischen und heterozyklischen Verbindungen eingesetzt werden, die beispielsweise als Antipyrene, bei der Synthese der Zwischenprodukte für Farbstoffe und Pigmente, bei der Synthese von Herbiziden, Wachstumregulatoren und auf anderen Gebieten Anwendung finden.

PATENTANSPRUCH:

Verfahren zur Herstellung von bromierten aromatischen und heterozyklischen die Akzeptor-Gruppen aufweisenden Verbindungen durch Behandlung der aromatischen und heterozyklischen Verbindungen, die Akzeptor-Gruppen enthalten, mit einem bromierenden Agens in Gegenwart von Schwefelsäure oder Oleum unter Anfallen eines Endproduktes, dadurch g e k e n n z e i c h n e t, dass man erfindungsgemäss als bromierende Agens Brom oder Salze der Bromwasserstoffsäure verwendet, wobei man den Prozess in Gegenwart der Schwefelsäure oder des Oleums in Kombination mit Salpetersäure oder ihren Salzen bei einer Temperatur von 20 bis I20°C bei folgenden Molverhältnissen der Reagenzien je I Mol der Ausgangsverbindung: Brom 0,5 bis 3,2 oder Salz der Bromwasserstoffsäure I,0 bis 7,0, Salpetersäure von 0,5 bis 3,0 oder ein Salz der Salpetersäure von I,0 bis 4,0, Schwefelsäure oder Oleum von 6,0 bis 70,0 durchführt.

# INTERNATIONAL SEARCH REPORT

International Application No PCT/SU 89/00333

**I. CLASSIFICATION OF SUBJECT MATTER** (if several classification symbols apply, indicate all) [6]

According to International Patent Classification (IPC) or to both National Classification and IPC

Int.Cl.$^5$ C07B 39/00, C07C I7/22

**II. FIELDS SEARCHED**

Minimum Documentation Searched [7]

| Classification System | Classification Symbols |
|---|---|
| Int.Cl.$^4$ | C07B 39/00, 9/00, C07C I7/22, 25/02, 25/13, 63/70, C07C 79/I2, 79/36, 79/46, I03/22, I43/40, I43/40, C07C I43/70, C07D 3II/88, C07/C 205/I2 |

Documentation Searched other than Minimum Documentation to the Extent that such Documents are Included in the Fields Searched [8]

**III. DOCUMENTS CONSIDERED TO BE RELEVANT** [9]

| Category * | Citation of Document, [11] with indication, where appropriate, of the relevant passages [12] | Relevant to Claim No [13] |
|---|---|---|
| X | JP,A, 56-30946 , 28 March 1981 (28.03.81) | In respect of obtaining bromo-benzoic accid |
| Y | -"-"-"-"-"-"-"-"-"-"-"-"-"-"-"-"-"-"-"-"-"- | I |
| X | JP,A, 56-30947, 28 March 1981 (28.03.81) | In respect of obtaining bromo-benzoic accid |
| Y | -"-"-"-"-"-"-"-"-"-"-"-"-"-"-"-"-"-"-"-"-"- | I |
| Y | SU,AI,I077874 (INSTITUT KHLORORGANICHESKOGO SINTEZA AN AzSSR), 7 March 1984 (07.03.84), see abstract | I |
| A | SU,AI, I203082 (MGU im.M.V.LOMONOSOVA) 7 January 1986 (07.01.86), see abstract | I |

* Special categories of cited documents: [10]

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step

"Y" document of particular relevance; the claimed invention cannot be considered to involve an inventive step when the document is combined with one or more other such documents, such combination being obvious to a person skilled in the art.

"&" document member of the same patent family

**IV. CERTIFICATION**

| Date of the Actual Completion of the International Search | Date of Mailing of this International Search Report |
|---|---|
| 12 March 1990 (12.03.90) | 02 April 1990 (02.04.90) |
| **International Searching Authority** | **Signature of Authorized Officer** |
| ISA/SU | |